# EUROPÄISCHE PATENTSCHRIFT

(11) **EP 3 225 169 B1**
(45) Veröffentlichungstag und Bekanntmachung des Hinweises auf die Patenterteilung: **03.07.2019**
(21) Anmeldenummer: 17157000.5
(22) Anmeldetag: 05.03.2014
(51) Int. Cl.: A61B 6/10, A61B 6/00, G21F 3/02

(54) **FAHRBARE STRAHLENSCHUTZANORDNUNG**
MOBILE RADIATION PROTECTION ASSEMBLY
AGENCEMENT MOBILE DE PROTECTION CONTRE LES RAYONNEMENTS

(30) Priorität: 06.03.2013 DE 102013203812
(43) Veröffentlichungstag der Anmeldung: 04.10.2017
(62) Teilanmeldung aus: 14714604.7
(73) Patentinhaber: MAVIG GmbH, 81829 München (DE)
(72) Erfinder: BALLSIEPER, Barbara, 81829 München (DE)
(74) Vertreter: Vossius & Partner Patentanwälte Rechtsanwälte mbB

(56) Entgegenhaltungen:
- WO-A1-2012/116030
- JP-Y1- S4 530 059
- US-A- 5 015 864
- US-A- 5 220 175
- US-A1- 2012 228 439

## Beschreibung

Die Erfindung betrifft eine fahrbare Strahlenschutzanordnung die einen oder mehrere flexible Strahlenschutzvorhänge aufweist. Diese fahrbare Strahlenschutzanordnung ist insbesondere geeignet zur Verwendung in der interventionellen Radiologie und bei entsprechenden Operationen. Die fahrbare Strahlenschutzanordnung dient zum Schutz von beteiligtem Personal wie dem Arzt oder Assistenten gegen auftretende Strahlung, insbesondere Röntgenstrahlung.

Aus der DE 203 07 606 U1 ist eine fahrbare Strahlenschutzanordnung mit transparenter Strahlenschutzscheibe bekannt, wobei die Strahlenschutzscheibe gewölbt ausgeführt und an einem doppelkreuzförmigen Fuß fixiert ist. Dabei sind die Arme des Fußes unterschiedlich lang ausgebildet um das Kipprisiko der Strahlenschutzvorrichtung zu vermindern. Die Strahlenschutzvorrichtung ist mit einer Höhenverstellung versehen und weist symmetrische oder asymmetrische Ausnehmungen in der Strahlenschutzscheibe auf, die vorgesehen sind um ein Durchgreifen der Strahlenschutzscheibe und damit einen größeren Aktionsradius und mehr Bewegungsfreiheit zu ermöglichen.

Die EP 0 345 548 A1 betrifft eine Strahlenschutzvorrichtung, die eine Röntgenstrahlen absorbierende Strahlenschutzwand aufweist, wobei die Strahlenschutzwand auf einem tragenden, frei fahrbaren Wagen angebracht ist. Des Weiteren weist die Strahlenschutzvorrichtung eine höhenverstellbare Halterung für zwei Wandelemente auf, wobei das erste Wandelement unterhalb des zweiten, durchsichtigen Wandelements angeordnet ist und wobei das zweite Wandelement um eine horizontale Achse schwenkbar ist.

Die US 5,015,864 beschreibt eine mobile Strahlenschutzvorrichtung, bei der ein Strahlenschutzanzug an einem fahrbaren Gestell angebracht ist.

Die US 3,308,297 betrifft eine fahrbare Strahlenschutzwand die in ihrer Mitte eine Aussparung mit einer geführten Schublade aufweist, so dass das behandelnde Personal durch diese Schublade durch die Strahlenschutzwand hindurchgreifen kann.

Der Erfindung liegt die Aufgabe zugrunde eine fahrbare Strahlenschutzanordnung bereitzustellen, die einfach und ermüdungsfrei von dem Fachpersonal während einer interventionellen Behandlung bewegt und verfahren werden kann.

Diese Aufgabe wird mit einer fahrbaren Strahlenschutzanordnung gemäß den Patentansprüchen gelöst.

Die erfindungsgemäße fahrbare Strahlenschutzanordnung eignet sich insbesondere zum einfachen Verfahren durch nur eine einzelne Person.

Die Erfindung geht von dem Grundgedanken aus, an einem zweiteiligen fahrbaren Gestell mehrere flexible Strahlenschutzvorhänge so anzubringen, dass das zweiteilige Gestell höhenverstellbar ist und sich die Strahlenschutzvorhänge in jeder Höhenposition überlappen und somit das Fachpersonal optimal vor Strahlung geschützt ist. Durch die Verwendung flexibler Strahlenschutzvorhänge wird im Gegensatz zu der Verwendung von starren Strahlenschutzwänden zudem das Gewicht der Strahlenschutzanordnung deutlich verringert. Gleichzeitig ist eine solche fahrbare Strahlenschutzanordnung in stehender oder sitzender Körperhaltung eines Benutzers jederzeit körpernah führbar. Durch die Überlappung der flexiblen Strahlenschutzvorhänge wird jederzeit sichergestellt, dass in einer beliebigen Höhenposition der fahrbaren Strahlenschutzanordnung das Bedienpersonal vor Strahlung geschützt ist.

Dabei hat die Erfindung den Vorteil, dass einer der flexiblen Strahlenschutzvorhänge vorzugsweise bis zu der Unterkante der Strahlenschutzanordnung reicht, so dass auch in diesem Bereich keine Strahlung das zu schützende Bedienpersonal erreichen kann. Gleichzeitig ermöglicht jedoch die Verwendung eines flexiblen Strahlenschutzvorhangs die gefahrlose Betätigung von Fußtastern der zu bedienenden medizinischen Instrumente. Aus diesem Grund ist das Fahrgestell der fahrbaren Strahlenschutzanordnung auch derart ausgestaltet, dass der Fuß des Bedienpersonals die Fußtaster der medizinischen Instrumente problemlos erreichen kann, das heißt, dass ein Durchgriff auf der gesamten Höhe des Fahrgestells ausgebildet ist. Durch diesen Durchgriff kann der Fuß des Bedienpersonals die Fußtaster ungehindert erreichen. Der Schutzvorhang muss allerdings durch seine Flexibilität dabei nicht geöffnet werden, sondern kann vor dem Fuß oder Bein vorgeschoben werden. Dabei sind der Fuß und das Bein bei der Betätigung der Fußtaster optimal vor Strahlung geschützt.

Die Erfindung betrifft eine fahrbare Strahlenschutzanordnung mit einem auf einer Seite offenem Gestell mit einem unteren Teil und einem oberen Teil, wobei das obere Teil gegenüber dem unteren Teil in der Höhe verstellbar ist, mindestens zwei flexiblen Strahlenschutzvorhängen, zum Schutz vor Strahlung, vorzugsweise Röntgenstrahlung, wobei ein erster der mindestens zwei Strahlenschutzvorhänge an dem unteren Teil des Gestells angebracht ist und sich bis zu der Unterkante des Gestells erstreckt. Des Weiteren ist ein zweiter der mindestens zwei Strahlenschutzvorhänge an dem oberen Teil des Gestells derart angebracht und ausgestaltet, dass sich die mindestens zwei Strahlenschutzvorhänge in jeder Höhenposition, in der das obere Teil gegenüber dem unteren Teil eingestellt ist, überlappen.

In einer weiteren Ausführungsform weist das untere Gestell ferner ein Fahrgestell auf, wobei das Fahrgestell mehrere Lenkrollen, beispielsweise vier Lenkrollen aufweisen kann.

Gemäß einer weiteren Ausführungsform ist das Fahrgestell derart ausgestaltet, dass an der Seite, die der offenen Seite des Gestells gegenüber liegt, ein Durchgriff auf der gesamten Höhe des Fahrgestells ausgebildet ist.

In einer weiteren Ausführungsform weist das Fahrgestell einen ersten und einen zweiten vorzugsweise bogenförmigen Ausleger auf, an dem die Lenkrollen befestigt sind. Der bogenförmige Ausleger kann dabei die Form eines Standfußes aufweisen, wobei das Fahrgestell nicht mit dem Standfuß direkt, sondern mit den an dem Standfuß befestigten Lenkrollen Bodenkontakt hat.

In einer weiteren Ausführungsform kann das untere Gestell ein erstes und ein zweites Seitenteil aufweisen. Dabei kann der erste Ausleger des Fahrgestells an dem ersten Seitenteil angebracht und der zweite Ausleger des Fahrgestells an dem zweiten Seitenteil angebracht sein.

Gemäß einer weiteren Ausführungsform weist das erste und das zweite Seitenteil erste und zweite Rohre in vertikaler Richtung auf, wobei die ersten und zweiten Rohre des ersten Seitenteils durch Querverbindungen miteinander verbunden werden können und die ersten und zweiten Rohre des zweiten Seitenteils durch Querverbindungen ebenfalls miteinander verbunden werden können.

In einer weiteren Ausführungsform sind zwischen den ersten und zweiten Rohren des jeweiligen ersten und zweiten Seitenteils Abdeckungen angebracht.

Gemäß einer weiteren Ausführungsform können das erste und das zweite Seitenteil mit einer Querverstrebung verbunden sein. Ausführungsgemäß befindet sich diese Querverbindung an der Seite eines Gestells, die der offenen Seite des Gestells gegenüber liegt.

Gemäß einer weiteren Ausführungsform weist das untere Teil ein vorzugsweise bügelförmiges Verbindungselement auf, welches an den ersten und zweiten Rohren angebracht ist. Dabei kann das vorzugsweise bügelförmige Verbindungselement an den oberen Kanten der ersten und zweiten Rohre angebracht sein, so dass es dieses nach oben hin abschließt. Erfindungsgemäß können an dem vorzugsweise bügelförmigen Verbindungselement auf der Innen- und auf der Außenseite Ösenknöpfe angebracht sein, an denen ein oder mehrere Strahlenschutzvorhänge befestigt werden. Somit können an der Innenseite des Gestells und/oder an der Außenseite des Gestells flexible Strahlenschutzvorhänge je nach Strahlendosis angebracht werden. Ein Strahlenschutzvorhang ist insbesondere dann flexibel, wenn er derart ausgestaltet ist, dass er sich aufgrund seiner Beschaffenheit an die äußere Form des Gestells anpasst und/oder durch einfache manuelle Manipulation in eine andere Form gebracht werden kann. Ein flexibler Strahlenschutzvorhang kann insbesondere leicht biegsam, nachgebend, anpassungsfähig und/oder elastisch sein.

Erfindungsgemäß kann das Verbindungselement die Form eines Halbkreises, eines an den Kanten abgerundeten halben Rechtecks oder eines halben Vielecks aufweisen. Die Form des Verbindungselementes ist jedoch nicht auf diese Formen beschränkt. So ist weiterhin beispielsweise ein Dreiviertelkreis oder ein Vollkreis möglich. Vor allem im Fall eines Dreiviertelkreises oder eines Vollkreises ist es vorteilhaft durch ein oder mehrere bewegliche und arretierbare Scharniere das Verbindungselement zu segmentieren. Durch ein Aufklappen des segmentierten Verbindungselements kann dem Verwender der fahrbaren Strahlenschutzanordnung der Einstieg in die Strahlenschutzanordnung erleichtert werden.

In einer weiteren Ausführungsform weist das obere Teil mehrere Tragstangen und das untere Teil mehrere Lagerbuchsen auf, wobei die Tragstangen des oberen Teils zur Verstellung der Höhenposition in jeweils einer zugehörigen Lagerbuchse hin und her beweglich sind. Des Weiteren kann das obere Teil ein vorzugsweise bogenförmiges Verbindungselement aufweisen. Auch ist in diesem Fall das bogenförmige Verbindungselement des oberen Teils nicht auf eine bestimmte Form beschränkt, sondern kann ebenfalls die Form eines Halbkreises, eines Dreiviertelkreises oder eines Vollkreises, eines an den Kanten abgerundeten halben Rechtecks oder eines halben Vielecks aufweisen. Im Fall einer oben beschriebenen Segmentierung des Verbindungselements des unteren Teils ist es ferner vorteilhaft, dass das Verbindungselement des oberen Teils ebenfalls eine entsprechende Segmentierung aufweist.

Gemäß einer weiteren Ausführungsform sind ein Teil der Lagerbuchsen in das Verbindungselement des unteren Teils und der andere Teil der Lagerbuchsen in die ersten und zweiten Rohre des unteren Teils eingepasst.

In einer weiteren Ausführungsform ist das bogenförmige Verbindungselement des oberen Teils an den Tragestangen angebracht. Dabei kann das vorzugsweise bogenförmige Verbindungselement des oberen Teils derart an dem oberen Ende der Tragstangen angebracht sein, dass es mit diesen bündig abschließt.

In einer weiteren Ausführungsform weist die fahrbare Strahlenschutzanordnung ein Gegengewicht oder eine Gegenfeder auf, die geeignet ist um die Gewichtskraft des oberen Teils und der an dem oberen Teil angebrachten Strahlenschutzvorhang/Strahlen-schutzvorhänge, der/die an dem oberen Teil des Gestells angebracht ist/sind auszugleichen. Dabei ist das Gegengewicht oder die Gegenfeder an dem unteren Teil des Fahrgestells angebracht und mit dem oberen Teil des Fahrgestells verbunden, beispielsweise durch einen Seilzug/ein Seilzugsystem. Idealerweise bewirkt das Gegengewicht oder die Gegenfeder, dass die Gewichtskraft des oberen Teils und des an dem oberen Teil angebrachten Strahlenschutzvorhangs/der Strahlenschutzvorhänge derart ausgeglichen ist, dass der obere Teil und die daran befindlichen Strahlenschutzvorhänge ohne weitere Fixierung in ihrer Position ohne weitere Hilfsmittel verharren.

Gemäß einer weiteren Ausführungsform weist das untere Teil eine Feststelleinrichtung auf, die geeignet ist mindestens einen der mehreren Tragstangen des oberen Teils zu fixieren, so dass das obere Teil in einer in das untere Teil eingeschobenen Position adaptiert wird. Erfindungsgemäß ist diese Feststelleinrichtung insbesondere anwendbar im Falle des Fehlens eines Gegengewichts oder einer Gegenfeder beziehungsweise einer nicht ausreichenden Tarierung des Gegengewichts bzw. der Gegenfeder.

In einer weiteren Ausführungsform weisen die flexiblen Strahlenschutzvorhänge mindestens ein Material auf, das ein Element mit einer Ordnungszahl von 50 - 83 enthält. Ein solches Element kann beispielsweise Zinn, Wolfram, Gadolinium, Antimon, Bismut, Blei oder Barium sein.

Ein Strahlenschutzvorhang kann in einer weiteren Ausführungsform ein Blei-Ersatzmaterial als leichtes Strahlenschutzmaterial für einen großen Energieanwendungsbereich aufweisen, wie es beispielsweise in DE 10 2004 001 328 A1 beschrieben wird. Desweiteren kann der Strahlenschutzvorhang ein geschichtetes Bleifrei-Röntgenschutzmaterial aus Einzelverbundschichten aufweisen, wie es in DE 10 2006 028 958 A1 offenbart ist. Auch kann der Strahlenschutzvorhang eine beschichtete Mikrofaserbahn gemäß DE 10 2009 037 565 A1 aufweisen.

Die Erfindung wird nachstehend anhand von Beispielen und der Zeichnungen näher erläutert.

Es zeigen:
Figur 1 eine perspektivische Ansicht einer fahrbaren Strahlenschutzanordnung ohne Strahlenschutzvorhänge gemäß einer ersten bevorzugten Ausführungsform der vorliegenden Erfindung,
Figur 2 eine perspektivische Ansicht des unteren Teils einer fahrbaren Strahlenschutzanordnung ohne Strahlenschutzvorhänge gemäß der ersten bevorzugten Ausführungsform,
Figur 3 eine Seitenansicht einer fahrbaren Strahlenschutzanordnung ohne Strahlenschutzvorhänge gemäß der ersten bevorzugten Ausführungsform der vorliegenden Erfindung,
Figur 4 eine Obenaufsicht einer fahrbaren Strahlenschutzanordnung ohne Strahlenschutzvorhänge gemäß der ersten Ausführungsform der vorliegenden Erfindung,
Figur 5 eine perspektivische Ansicht einer fahrbaren Strahlenschutzanordnung ohne Strahlenschutzvorhänge gemäß einer zweiten bevorzugten Ausführungsform der vorliegenden Erfindung,
Figur 6a eine perspektivische Außenansicht des unteren Teils einer fahrbaren Strahlenschutzanordnung mit Strahlenschutzvorhängen gemäß der ersten bevorzugten Ausführungsform,
Figur 6b eine perspektivische Innenansicht des unteren Teils einer fahrbaren Strahlenschutzanordnung mit Strahlenschutzvorhängen gemäß der ersten bevorzugten Ausführungsform,
Figur 7a eine perspektivische Außenansicht einer fahrbaren Strahlenschutzanordnung mit Strahlenschutzvorhängen gemäß der ersten bevorzugten Ausführungsform, und
Figur 7b eine perspektivische Innenansicht einer fahrbaren Strahlenschutzanordnung mit Strahlenschutzvorhängen gemäß der ersten bevorzugten Ausführungsform.

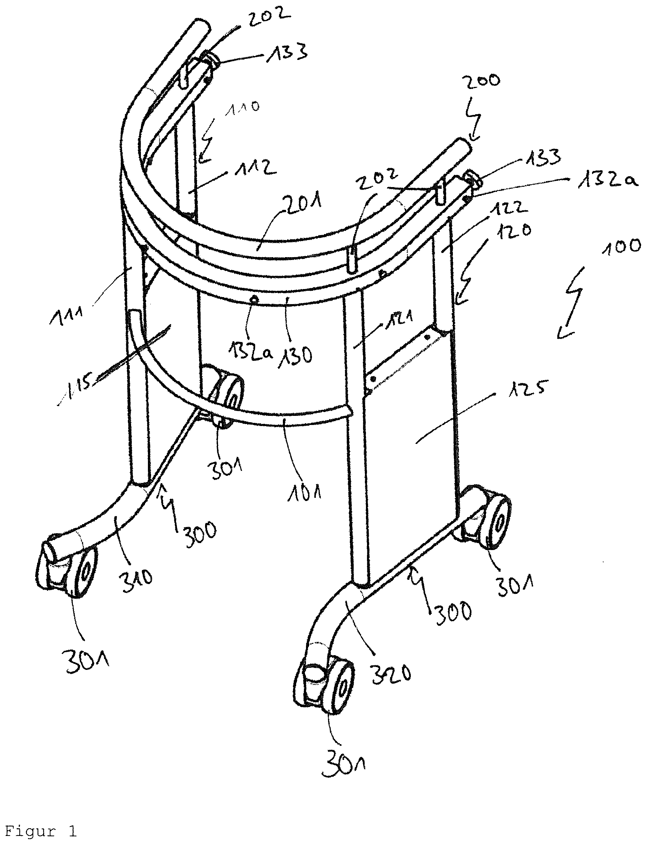

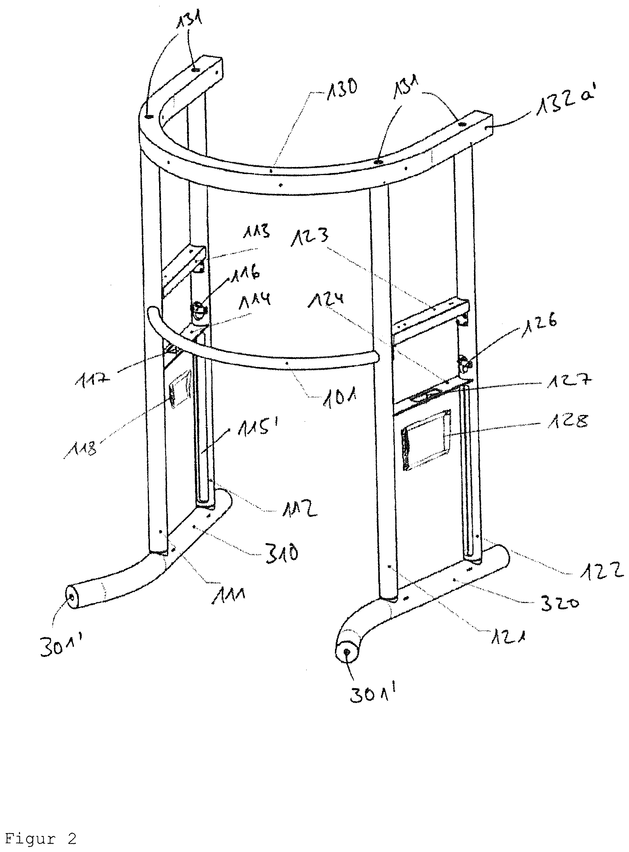

Figur 1 zeigt eine perspektivische Ansicht einer fahrbaren Strahlenschutzanordnung gemäß einer ersten Ausführungsform der vorliegenden Erfindung. Aus Gründen der Übersichtlichkeit wurden die Strahlenschutzvorhänge nicht gezeigt. Das Gestell der fahrbaren Strahlenschutzanordnung weist ein unteres Teil 100 auf. Das untere Teil 100 weist wiederum ein erstes 110 und ein zweites 120 Seitenteil auf. Grundgerüst des ersten 110 und zweiten 120 Seitenteils sind jeweils ein erstes 111, 121 und zweites Rohr 112, 122, wobei die ersten und zweiten Rohre 111, 121, 112, 122 sich in vertikaler Richtung erstrecken und wobei die ersten 111, 112 mit den zweiten 112, 122 Rohren durch erste 113, 123 und zweite 114, 124 Querverbindungen (siehe Figur 2) miteinander verbunden sind. Das erste 110 und zweite 120 Seitenteil sind ungefähr auf halber bis zwei Drittel der Höhe der Seitenteile mit einer Querverstrebung 101 miteinander verbunden. Dabei ist die Querverstrebung an den jeweiligen ersten Rohren 111, 121 des ersten 110 und zweiten 120 Seitenteils angebracht. Somit bildet sich zwischen den zweiten Rohren 112, 122 eine offene Seite. Die Querverstrebung 101 ist dabei so hoch angebracht, dass ein Fuß oder ein Bein des Bedienpersonals der/das bequem nach vorne in Fluchtrichtung hin ausgestreckt wird, um einen Fußtaster zu erreichen, nicht behindert wird.

Zwischen den ersten 111, 121 und zweiten 112, 122 Rohren befinden sich Abdeckplatten 115, 125, die den Zwischenraum zwischen den ersten 111, 121 und zweiten 112, 122 Rohren abdecken.

Das untere Teil 100 weist ferner ein Fahrgestell 300 auf, welches ausführungsgemäß zwei Ausleger 310 und 320 besitzt. Jeder Ausleger 310, 320 hat die Form eines Standfußes der jeweils in Fluchtrichtung nach außen gewölbt ist. An dem jeweiligen Ausleger sind Lenkrollen 301 angebracht so dass untere Teil 100 auf den Lenkrollen aufsitzt. Die Lenkrollen 301 befinden sich dabei an den äußersten Enden des ersten 310 und zweiten 320 Ausleger in entsprechenden Aufnahmen 301'. Die Aufnahmen 301' für die Lenkrolle sind in Figur 2 gezeigt. Der erste Ausleger 310 wird an dem ersten Seitenteil 110 angebracht, in dem es mit dem ersten 111 und zweiten 112 Rohr des ersten Seitenteils 110 verbunden wird. Analog dazu wird der zweite Ausleger 320 an dem zweiten Seitenteil 120 angebracht. Die Verbindung der ersten 111, 121 und jeweiligen zweiten 112, 122 Rohre mit den jeweiligen Ausleger 310 bzw. 320 kann durch eine Schraub- oder Schweißverbindung erfolgen.

Die oberen Enden der ersten 111, 121 und zweiten 112, 122 Rohre sind mit einem Verbindungselement 130 verbunden. Dieses Verbindungselement 130 hat ausführungsgemäß die Form eines halbkreisförmigen Bügels mit rechteckigem Durchmesser. Das Verbindungselement 130 schließt die ersten 111, 121 und zweiten 112, 122 Seitenrohre nach oben hin bündig ab. An der Außen- und Innenkante des Verbindungselements 130 befinden sich Ösenknöpfe 132a bzw. 132i zur Anbringung von Strahlenschutzvorhängen an der Außen- und/oder an der Innenseite des unteren Gestells 100 der fahrbaren Strahlenschutzanordnung. Die Anbringung der Vorhänge wird weiter unten eingehender erläutert.

In das Verbindungselement 130 sind zusätzlich vier Lagerbuchsen 131 in entsprechende Bohrungen eingelassen. Die Bohrungen sind dabei derart ausgestaltet, dass sie durch das Verbindungselement durchgehen, so dass ein Durchgang durch das Verbindungselement 130 zu den ersten und zweiten Rohren 111, 121, 112, 122 besteht.

In den ersten und zweiten Rohren 111, 121, 112, 122 befinden sich zweite Lagerbuchsen 116, 126 in einem gewissen Abstand zu den ersten Lagerbuchsen 131. Dieser Abstand liegt ausführungsgemäß in einem Bereich zwischen 30 cm und 40 cm, bevorzugt beträgt er etwa 35,4 cm.

Das obere Teil 200 weist vier Tragstangen 202 auf, die an ihrem oberen Ende ebenfalls mit einem Verbindungselement 201 verbunden sind. Das Verbindungselement 201 weist dabei ebenfalls eine halbkreisförmige Bügelform allerdings mit rundem Querschnitt auf. Ausführungsgemäß schließt dabei das Verbindungselement 201 die Tragstangen 202 nach oben hin bündig ab.

Die Lagerbuchsen 131, 116, 126 des unteren Teils 100 dienen nun dazu, die Tragstangen 202 des oberen Teils 200 aufzunehmen, so dass die Tragstangen 202 in die Lagerbuchsen und somit in die Rohre 111, 121, 112, 122 eingefahren werden können. Dazu haben ausführungsgemäß die Lagerbuchsen 131, 116, 126 einen Innendurchmesser, der bis auf eine gewisse Toleranz dem Außendurchmesser der Tragstangen 202 entspricht. Dadurch wird eine kontinuierlich variable Höhenverstellbarkeit des oberen Teils 200 erreicht.

Die Höhenposition des zweiten Teils 200 kann durch eine oder mehrere Feststelleinrichtungen 133, die an dem Verbindungselement 130 des unteren Teils 100 angebracht sind, zusätzlich fixiert werden. Für die Fixierung der Höhenposition des zweiten Teils 200 durch die Feststelleinrichtung 133 wird eine Schraube, z.B. eine Handschraube in ein Gewinde eingedreht, das sich senkrecht zu der Längsrichtung der Bohrung befindet und von einer Außenseite des Verbindungselements bis zu einer der Bohrungen erstreckt, in die auch die Lagerbuchsen 131 eingelassen sind. Das Gewinde reicht dabei bis zu der Innenseite der Bohrung, so dass durch das Ende der eingeführten Schraube eine der Tragstangen 202 des oberen Teils in ihrer Höhenposition fixiert werden kann. Ausführungsgemäß sind zwei solcher Feststelleinrichtungen 133 an den jeweiligen Enden des Verbindungselements 130 angebracht.

Wie bereits erläutert befinden sich zur Befestigung von Strahlenschutzvorhängen an dem unteren Teil 100 Ösenknöpfe 132a bzw. 132i sowohl innen als auch außen an dem Verbindungselement 133. Je nach Bedarf, d. h. je nach der abzuschirmenden Strahlenbelastung können ein oder mehrere Strahlenschutzvorhänge sowohl innen als auch außen an dem unteren Teil 100 der fahrbaren Strahlenschutzanordnung angehängt werden.

An dem oberen Teil 200 können ein oder mehrere Strahlenschutzvorhänge über das Verbindungselement 201 als Überwurf angebracht sein, wobei sich ein Teil der einen oder mehreren Strahlenschutzvorhänge auf der Vorderseite der fahrbaren Strahlenschutzanordnung, der andere Teil an der Rückseite der fahrbaren Strahlenschutzanordnung erstreckt. Seitlich werden die ein oder mehreren Strahlenschutzvorhänge beispielsweise durch Knöpfe oder Klettverschlüsse verschlossen.

Figur 2 zeigt eine perspektivische Ansicht des unteren Teils 100 der fahrbaren Strahlenschutzanordnung ohne Strahlenschutzvorhänge gemäß der ersten bevorzugten Ausführungsform.

Deutlich erkennbar ist an dem Fahrgestell 300 des unteren Teils 100 an dem ersten 310 und dem zweiten 320 Ausleger die Aufnahmen 301' für die entsprechenden Lenkrollen 301.

Ferner sind an dem unteren Teil 100 erste 113, 123 und zweite 114, 124 Querverbindungen erkennbar mit denen die ersten 111, 121 und zweiten 112, 122 Rohre des ersten 110 Seitenteils bzw. des zweiten 120 Seitenteils aus Stabilitätsgründen miteinander verbunden werden können. Die zweiten Querverbindungen 114, 124 weisen dabei Aussparungen 117, 127 auf. Durch diese Aussparungen führt ein Seilzug bzw. ein Seilzugsystem (nicht dargestellt), welcher/welches das obere Teil 200 mit einem Gegengewicht bzw. einer Gegenfeder 128 verbindet. Das Gegengewicht/die Gegenfeder 128 ist dabei idealerweise so austariert, dass es die Gewichtskraft des oberen Teils 200 und der an dem oberen Teil 200 angebrachten Strahlenschutzvorhang bzw. Strahlenschutzvorhänge, der/die an dem oberen Teil des Gestells angebracht ist/sind ausgleicht. Im Idealfall sorgt dieser Ausgleich dafür, dass der obere Teil 200 und die daran befindlichen Strahlenschutzvorhänge ohne weitere Fixierung, in ihrer Position ohne weitere Hilfsmittel verharren. Vorzugsweise wird auch bei optimaler Austarierung ausführungsgemäß eine Feststelleinrichtung 133 bzw. mehrere Feststelleinrichtungen 133 angebracht. Dadurch ist eine Fixierung des oberen Teils 100 am unteren Teil 200 möglich, wobei die Gesamthöhe vorzugsweise in Stufen oder stufenlos einstellbar ist.

Wie in Figur 2 dargestellt, sind die zweiten Lagerbuchsen 116, 126 in den ersten 111, 121 sowie zweiten 112, 122 Rohren in einem bestimmten Abstand zum Verbindungselement 130 des unteren Teils 100 angebracht. In dem Verbindungselement 130 sind zudem die Bohrungen 132a' für die äußeren Ösenknöpfe 132a erkennbar.

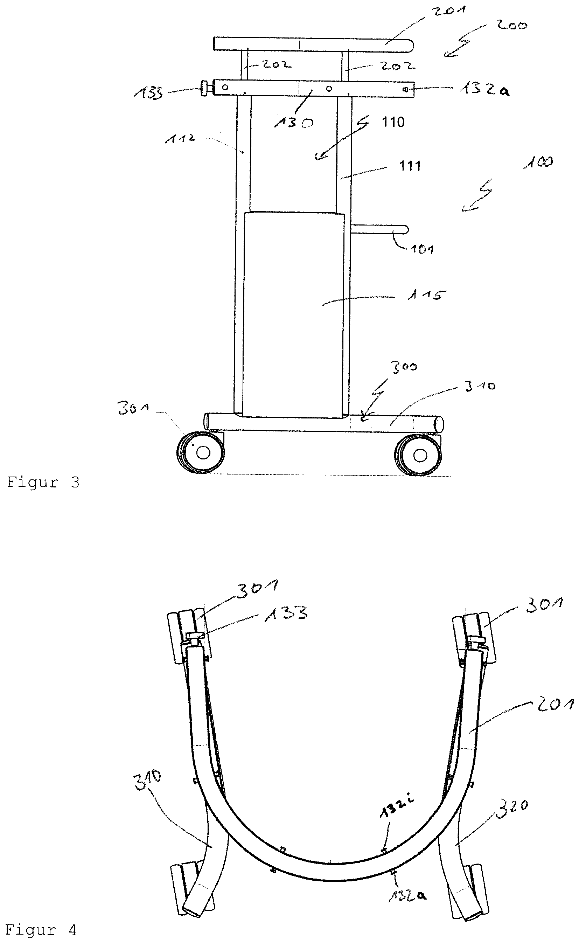

Figur 3 zeigt eine Seitenansicht der fahrbaren Strahlenschutzanordnung ohne Strahlenschutzvorhänge gemäß der ersten bevorzugten Ausführung der vorliegenden Erfindung, sowie Figur 4 eine Obenaufsicht der fahrbaren Strahlenschutzanordnung ohne Strahlenschutzvorhänge.

Figur 3 zeigt dabei das obere Teil 200 mit dem Verbindungselement 201 und den Tragstangen 202. Außerdem zeigt Figur 3 das untere Element 100 mit dem Fahrgestell 300, sowie das erste Seitenteil 110 mit dem ersten Rohr 111 und dem zweiten Rohr 112 sowie der ersten Abdeckung 115. Erkennbar ist zudem das Verbindungselement 130 mit den inneren Ösenknöpfen 132i und der Feststelleinrichtung 133. Figur 3 zeigt außerdem die Querverstrebung 101, die das erste Seitenteil 110 mit dem zweiten Seitenteil 120 verbindet.

Die Obenaufsicht der Figur 4 zeigt insbesondere die außen und innen liegenden Ösenknöpfe 132a bzw. 132i des unteren Teils 100 an denen ein oder mehrere Strahlenschutzvorhänge angebracht werden können.

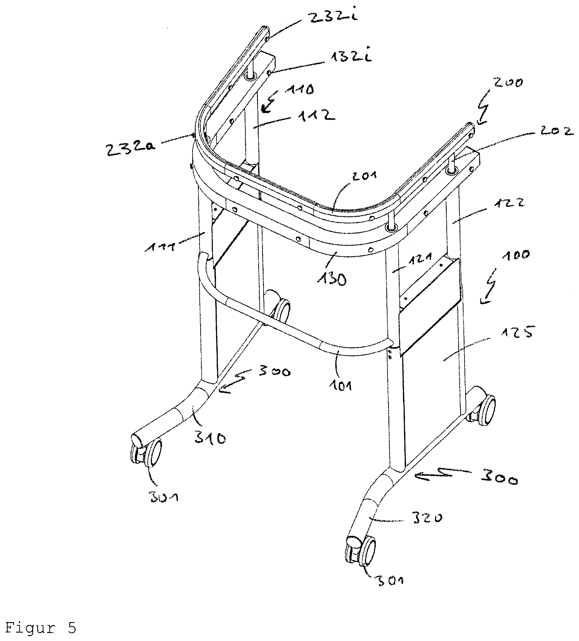

Figur 5 zeigt eine perspektivische Ansicht einer fahrbaren Strahlenschutzanordnung ohne Strahlenschutzvorhänge gemäß einer zweiten bevorzugten Ausführungsform der vorliegenden Erfindung.

Die Verbindungselemente 130 des unteren Teils 100 sowie 201 des oberen Teils 200 sind dabei nicht wie in der ersten Ausführungsform in Form eines halbkreisförmigen Bügels ausgebildet, sondern eines rechteckigen Bügels mit abgerundeten Kanten.

Ein weiterer Unterschied zur ersten Ausführungsform ist dass das obere Teil 200 ebenfalls äußere Ösenknöpfe 232a und innere Ösenknöpfe 232i an der Außen- wie an der Innenseite zur Anbringung von einem oder mehreren Strahlenschutzvorhängen aufweist. So kann auch an dem oberen Teil je nach Bedarf, das heißt je nach Strahlendosis ein oder mehrere Strahlenschutzvorhänge befestigt werden.

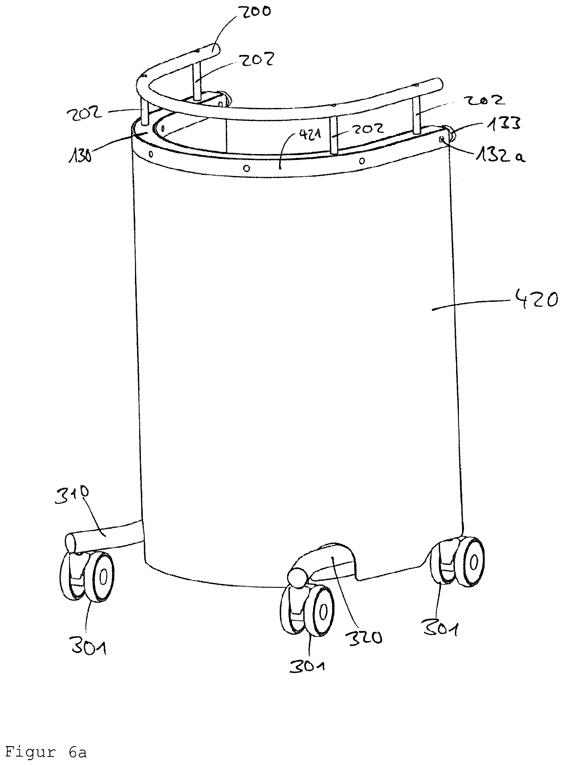

Figur 6a zeigt eine perspektivische Außenansicht des unteren Teils 100 einer fahrbaren Strahlenschutzanordnung mit Strahlenschutzvorhängen gemäß der ersten bevorzugten Ausführungsform.

Ein äußerer unterer Strahlenschutzvorhang 420 ist mit Hilfe einer äußeren Ösenleiste 421 mit entsprechenden Ösen an den äußeren Ösenknöpfen 132a des unteren Teils 100 befestigt.

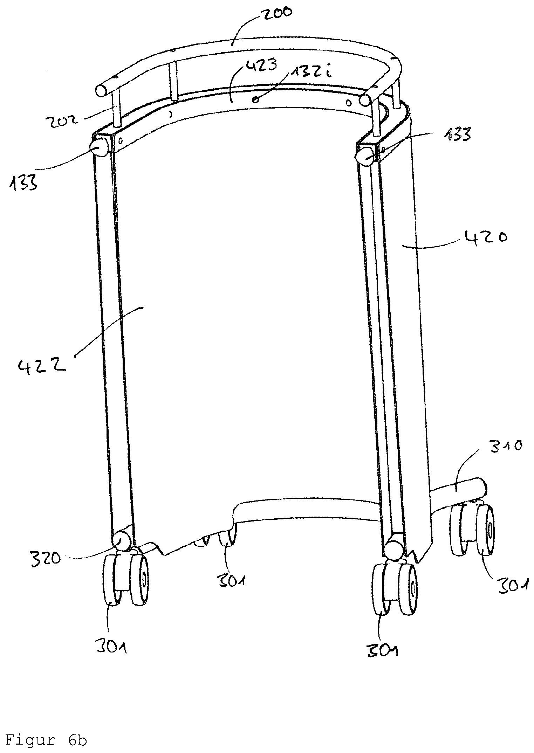

Figur 6b zeigt eine perspektivische Innenansicht des unteren Teils 100 einer fahrbaren Strahlenschutzanordnung mit Strahlenschutzvorhängen gemäß der ersten bevorzugten Ausführungsform.

Es ist deutlich zu erkennen, dass an den inneren Ösenknöpfen 132i des unteren Teils 100 ein innerer unterer Strahlenschutzvorhang 422 mit Hilfe einer inneren Ösenleiste 423 befestigt werden kann.

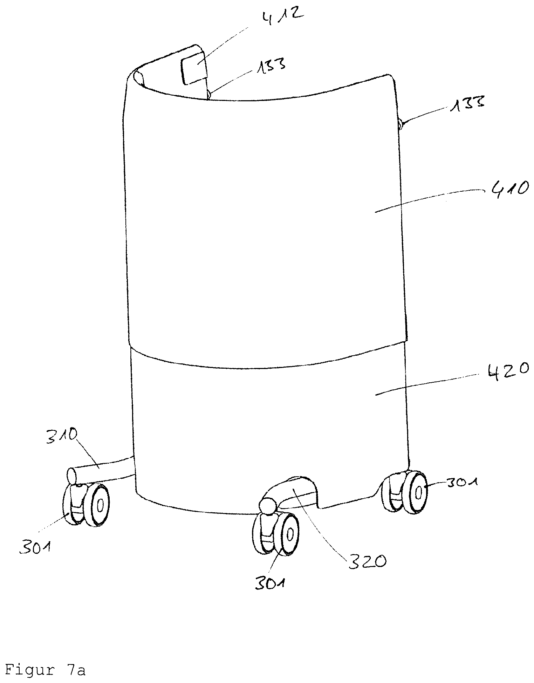

Figur 7a zeigt eine perspektivische Außenansicht einer fahrbaren Strahlenschutzanordnung mit Strahlenschutzvorhängen gemäß der ersten bevorzugten Ausführungsform, bei der das obere Teil 200 in das untere Teil 100 eingesteckt wurde.

Der obere Strahlenschutzvorhang 410 ist als Überwurf über das Verbindungselement 201 des oberen Teils 200 derart ausgestaltet, dass sowohl die Vorderseite als auch die Rückseite der fahrbaren Strahlenschutzanordnung bis zu einem bestimmten Punkt über dem Fahrgestell 300 abgedeckt wird. Gemäß der ersten Ausführungsform wird die Strahlenschutzanordnung sowohl auf der Vorder- als auch auf der Rückseite bis zu einem Bereich von 40 cm bis 55 cm über dem Fahrgestell, bevorzugt bis zu 47 cm über dem Fahrgestell, von dem oberen Strahlenschutzvorhang 410 abgedeckt. An den beiden freien oberen und unteren freien Enden ist jeweils ein Verbindungsstreifen 412 angeordnet, mit dem der die Vorderseite der fahrbaren Strahlenschutzanordnung abdeckende Teil des oberen Strahlenschutzvorhangs 410 und der die Rückseite der fahrbaren Strahlenschutzanordnung abdeckende Teil des oberen Strahlenschutzvorhangs 410 miteinander lösbar verbunden werden. Ausführungsgemäß weisen diese Verbindungsstreifen 412 jeweils einen ersten Klettstreifen mit einer Widerhakenseite auf, der mit einem zweiten Klettstreifen mit einer Schlaufenseite verbindbar ist, wobei der zweite Klettstreifen an dem Teil angeordnet ist, der die Rückseite der fahrbaren Strahlenschutzanordnung abdeckt. Diese Anordnung kann auch umgekehrt werden.

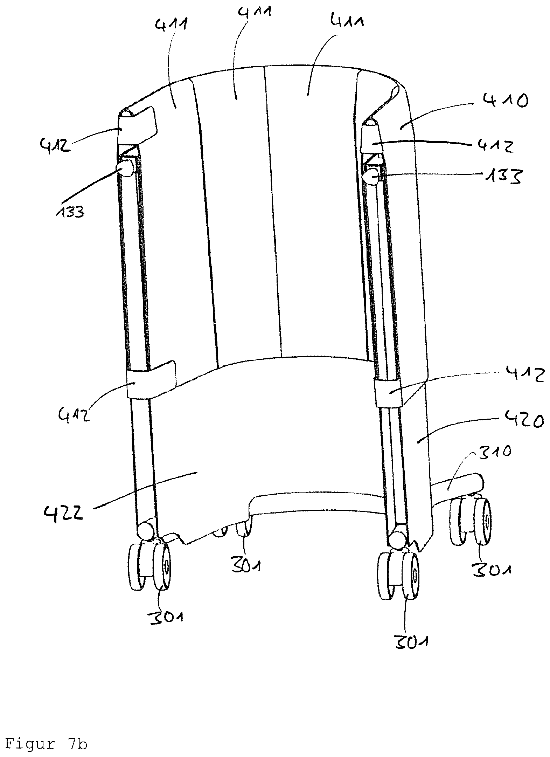

Figur 7b zeigt eine perspektivische Innenansicht einer fahrbaren Strahlenschutzanordnung mit Strahlenschutzvorhängen gemäß der ersten bevorzugten Ausführungsform.

Ausführungsgemäß ist der die Rückseite der fahrbaren Strahlenschutzanordnung abdeckende Teil des oberen Strahlenschutzvorhangs 410 in einzelne geschlitzte Segmente 411 unterteilt. Durch die Schlitzung des die Rückseite der fahrbaren Strahlenschutzanordnung abdeckenden Teils des oberen Strahlenschutzvorhangs 410 in einzelne Segmente 411 kann der obere Strahlenschutzvorhang 410 an die gebogene Form der fahrbaren Strahlenschutzanordnung einfach angepasst werden. Die Segmente 411 sind dabei so dimensioniert, dass sich ihre Kanten teilweise überlappen, so dass keine Strahlung an den Bereichen der Schlitze durchdringen kann. Es werden insgesamt vier Verbindungsstreifen 412 - jeweils zwei an einer Seite, d.h. ein Verbindungsstreifen 412 oben und unten an dem einem freien Ende und an dem gegenüberliegenden freien Ende - zum Verbinden des vorderen und des hinteren Teils des oberen Strahlenschutzvorhangs 410 verwendet.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Es versteht sich, dass Fachleute Änderungen und Abwandlungen machen können, ohne den Umfang und den Geist der folgenden Ansprüche zu verlassen. Insbesondere umfasst die Erfindung ebenfalls Ausführungsformen mit jeglicher Kombination von Merkmalen, die vorstehend oder nachfolgend zu verschiedenen Ausführungsformen genannt oder gezeigt sind.

Die Erfindung umfasst ebenfalls einzelne Merkmale in den Figuren auch wenn sie dort im Zusammenhang mit anderen Merkmalen gezeigt sind und/oder vorstehend oder nachfolgend nicht genannt sind. Auch können die in den Figuren und der Beschreibung beschriebenen Alternativen von Ausführungsformen und einzelne Alternativen deren Merkmale vom Erfindungsgegenstand beziehungsweise von den offenbarten Gegenständen ausgeschlossen sein. Die Offenbarung umfasst Ausführungsformen, die ausschließlich die in den Ansprüchen beziehungsweise in den Ausführungsbeispielen beschriebenen Merkmale umfasst sowie auch solche, die zusätzliche andere Merkmale umfassen.

## Patentansprüche

1. Fahrbare Strahlenschutzanordnung mit:
einem auf einer Seite offenen Gestell mit einem unteren Teil (100) und einem oberen Teil (200), wobei das obere Teil (200) gegenüber dem unteren Teil (100) in der Höhe verstellbar ist,
wobei das untere Teil (100) des Gestells ein Fahrgestell (300) aufweist,
mindestens zwei flexiblen Strahlenschutzvorhängen, zum Schutz vor Strahlung, vorzugsweise Röntgenstrahlung,
wobei ein erster der mindestens zwei Strahlenschutzvorhänge an dem unteren Teil (100) des Gestells angebracht ist und sich vorzugsweise bis zu der Unterkante des Gestells erstreckt,
wobei ein zweiter der mindestens zwei Strahlenschutzvorhänge an dem oberen Teil (200) des Gestells derart angebracht und ausgestaltet ist, dass sich die mindestens zwei Strahlenschutzvorhänge in jeder Höhenposition, in der das obere Teil (200) gegenüber dem unteren Teil (100) eingestellt ist, überlappen,
wobei das obere Teil (200) ein bogenförmiges Verbindungselement (201) aufweist,
wobei das bogenförmige Verbindungselement (201) des oberen Teils (200) die Form eines Halbkreises oder eines an den Kanten abgerundeten halben Rechtecks aufweist,
**dadurch gekennzeichnet, dass** der zweite Strahlenschutzvorhang an dem oberen Teil (200) über das Verbindungselement (201) als Überwurf angebracht ist, so dass sowohl die Vorderseite als auch die Rückseite der fahrbaren Strahlenschutzanordnung bis zu einem bestimmten Punkt über dem Fahrgestell (300) abgedeckt wird.

2. Strahlenschutzanordnung nach Anspruch 1, wobei das Fahrgestell (300) einen ersten (310) und einen zweiten (320) Ausleger aufweist, an denen mehrere Lenkrollen (301) befestigt sind, wobei die Ausleger (310, 320) bevorzugt bogenförmig sind.

3. Strahlenschutzanordnung nach Anspruch 1 oder 2, wobei das Fahrgestell (300) derart ausgestaltet ist, dass an der Seite, die der offenen Seite des Gestells gegenüberliegt, ein Durchgriff auf der gesamten Höhe des Fahrgestells (300) ausgebildet ist.

4. Strahlenschutzanordnung nach einem der Ansprüche 1 bis 3, wobei der erste (310) und der zweite (320) Ausleger die Form eines Standfußes aufweisen und jeweils in Fluchtrichtung nach außen gewölbt sind.

5. Strahlenschutzanordnung nach einem der Ansprüche 1 bis 4, wobei das untere Gestell (100) ein erstes (110) und ein zweites Seitenteil (120) aufweist, wobei vorzugsweise der erste Ausleger (310) an dem ersten Seitenteil (110) angebracht ist und der zweite Ausleger (320) an dem zweiten Seitenteil (120) angebracht ist.

6. Strahlenschutzanordnung nach Anspruch 5, wobei das erste (110) und das zweite (120) Seitenteil mit einer Querverstrebung (101) verbunden sind.

7. Strahlenschutzanordnung nach einem der Ansprüche 1 bis 6, wobei das obere Teil (200) mehrere Tragstangen (202) und das untere Teil (100) mehrere Lagerbuchsen (116, 126, 131) aufweist, wobei die Tragstangen (202) des oberen Teils (200) zur Verstellung der Höhenposition in jeweils einer zugehörigen Lagerbuchse hin- und her beweglich sind.

8. Strahlenschutzanordnung nach Anspruch 7, wobei ein Teil der Lagerbuchsen in das Verbindungselement (130) des unteren Teils (100) und der andere Teil der Lagerbuchsen (116, 126) in erste (111, 121) und zweite Rohre (112, 122) eingepasst sind.

9. Strahlenschutzanordnung nach einem der Ansprüche 1 bis 8, wobei das vorzugsweise bogenförmige Verbindungselement (201) des oberen Teils (200) an den Tragstangen (202) angebracht ist.

10. Strahlenschutzanordnung nach einem der Ansprüche 7 bis 9, wobei das untere Teil (100) ferner eine Feststelleinrichtung (133) aufweist, die geeignet ist mindestens einen der mehreren Tragstangen (202) des oberen Teils (200) zu fixieren, so dass das obere Teil (200) in einer in das untere Teil (100) eingeschoben Position arretiert ist.

11. Strahlenschutzanordnung nach einem der Ansprüche 1 bis 10, wobei der zweite Strahlenschutzvorhang seitlich durch Knöpfe oder Klettverschlüsse verschlossen wird.

12. Strahlenschutzanordnung nach einem der Ansprüche 1 bis 11, wobei der zweite Strahlenschutzvorhang die Vorderseite der fahrbaren Strahlenschutzanordnung und die Rückseite der fahrbaren Strahlenschutzanordnung bis zu einem Bereich von 40 cm bis 55 cm über dem Fahrgestell (300), besonders bevorzugt bis zu 47 cm über dem Fahrgestell (300) abdeckt.

## Claims

1. Movable radiation protection system comprising:
a frame which is open on one side and which comprises a lower part (100) and an upper part (200), said upper part (200) being vertically adjustable relative to the lower part (100), wherein the lower part (100) of the frame comprises a chassis (300),
at least two flexible radiation protection drapes for protecting against radiation, preferably X-ray radiation,
wherein a first of the at least two radiation protection drapes is attached to the lower part (100) of the frame and extends preferably to the lower edge of the frame,
wherein a second of the at least two radiation protection drapes is attached to the upper part (200) of the frame and is formed such that the at least two radiation protection drapes overlap in each height position in which the upper part (200) is adjusted relative to the lower part (100),
wherein the upper part (200) comprises an arcuate connecting element (201),
wherein the arcuate connecting element (201) of the upper part (200) has the shape of a semicircle or a half rectangle with rounded edges,
**characterized in that** the second radiation protection drape is attached to the upper part (200) by means of the connecting element (201) as cover, so that both the front side and the rear side of the movable radiation protection system are covered up to a specific point above the chassis (300).

2. Radiation protection system according to claim 1, wherein the chassis (300) comprises a first cantilever arm (310) and a second cantilever arm (320) on which a plurality of steering rollers (301) are mounted, wherein the cantilever arms (310, 320) are preferably arcuate.

3. Radiation protection system according to claim 1 or 2, wherein the chassis (300) is designed such that a passage is formed over the entire height of the chassis (300) at the side opposite to the open side of the frame.

4. Radiation protection system according to any one of claims 1 to 3, wherein the first cantilever arm (310) and the second cantilever arm (320) have the shape of a foot and are each curved outwardly in the direction of escape.

5. Radiation protection system according to any one of claims 1 to 4, wherein the lower frame (100) comprises a first side part (110) and a second side part (120), wherein preferably the first cantilever arm (310) is attached to the first side part (110) and the second cantilever arm (320) is attached to the second side part (120).

6. Radiation protection system according to claim 5, wherein the first side part (110) and the second side part (120) are connected by a crossbar (101).

7. Radiation protection system according to any one of claims 1 to 6, wherein the upper part (200) comprises a plurality of supporting rods (202) and the lower part (100) comprises a plurality of bearing bushes (116, 126, 131), wherein the supporting rods (202) of the upper part (200) are each movable back and forth in an associated bearing bush for adjusting the height position.

8. Radiation protection system according to claim 7, wherein a part of the bearing bushes is fitted in the connecting element (130) of the lower part (100) and the other part of the bearing bushes (116, 126) is fitted in the first tubes (111, 121) and the second tubes (112, 122).

9. Radiation protection system according to any one of claims 1 to 8, wherein the preferably arcuate connecting element (201) of the upper part (200) is attached to the supporting rods (202).

10. Radiation protection system according to any one of claims 7 to 9, wherein the lower part (100) further comprises a retaining device (133) which is suitable for fixing at least one of the plurality of supporting rods (202) of the upper part (200) so that the upper part (200) is locked in a position in which it is inserted in the lower part (100).

11. Radiation protection system according to any one of claims 1 to 10, wherein the second radiation protection drape is closed at the side with buttons or hook-and-loop fasteners.

12. Radiation protection system according to any one of claims 1 to 11, wherein the second radiation protection drape covers the front side of the movable radiation protection system and the rear side of the movable radiation protection system up to an area of 40 cm to 55 cm above the chassis (300), particularly preferably up to 47 cm above the chassis (300).

## Revendications

1. Ensemble mobile de protection contre les radiations, comprenant :
une structure ouverte d'un côté avec une partie inférieure (100) et une partie supérieure (200), la partie supérieure (200) étant réglable en hauteur par rapport à la partie inférieure (100), la partie inférieure (100) de la structure comprenant une structure de châssis de roulement (300),
au moins deux rideaux flexibles de protection contre les radiations, destinés à assurer la protection à l'encontre de radiations, de préférence des rayons X, la configuration étant la suivante :
un premier desdits au moins deux rideaux de protection contre les radiations est placé sur la partie inférieure (100) de la structure et s'étend de préférence jusqu'au bord inférieur de la structure,
un deuxième desdits au moins deux rideaux de protection contre les radiations est placé sur la partie supérieure (200) de la structure et est configuré de manière telle, que lesdits au moins deux rideaux de protection contre les radiations se chevauchent mutuellement pour chaque position de hauteur à laquelle est réglée la partie supérieure (200) par rapport à la partie inférieure (100),
la partie supérieure (200) comporte un élément de liaison (201) cintré en forme d'arc, et
l'élément de liaison (201) cintré en forme d'arc de la partie supérieure (200) présente la forme d'un demi-cercle ou d'un demi-rectangle à sommets arrondis, **caractérisé**
**en ce que** le deuxième rideau de protection contre les radiations est placé sur la partie supérieure (200), par-dessus l'élément de liaison (201), en tant que coiffe, de manière telle, qu'aussi bien le côté avant que le côté arrière de l'ensemble mobile de protection contre les radiations, soient recouverts jusqu'à un certain niveau au-dessus de la structure de châssis de roulement (300).

2. Ensemble de protection contre les radiations selon la revendication 1, dans lequel la structure de châssis de roulement (300) comporte un premier (310) et un deuxième (320) avant-bras, sur lesquels sont fixées plusieurs roulettes directionnelles (301), les avant-bras (310, 320) étant de préférence de forme courbe.

3. Ensemble de protection contre les radiations selon la revendication 1 ou la revendication 2, dans lequel la structure de châssis de roulement (300) est configurée de manière telle, que sur le côté, qui est opposé au côté ouvert de la structure, soit formé un passage sur la totalité de la hauteur de la structure de châssis de roulement (300).

4. Ensemble de protection contre les radiations selon l'une des revendications 1 à 3, dans lequel le premier (310) et le deuxième (320) avant-bras présentent la forme d'un pied d'appui et sont respectivement courbés vers l'extérieur dans la direction d'alignement.

5. Ensemble de protection contre les radiations selon l'une des revendications 1 à 4, dans lequel la structure inférieure (100) présente une première (110) et une deuxième partie latérale (120), le premier avant-bras (310) étant de préférence agencé sur la première partie latérale (110) et le deuxième avant-bras (320) sur la deuxième partie latérale (120).

6. Ensemble de protection contre les radiations selon la revendication 5, dans lequel la première (110) et la deuxième partie latérale (120) sont reliées l'une à l'autre au moyen d'une traverse de renfort (101).

7. Ensemble de protection contre les radiations selon l'une des revendications 1 à 6, dans lequel la partie supérieure (200) comporte plusieurs tiges porteuses (202) et la partie inférieure (100) comporte plusieurs coussinets de palier (116, 126, 131), les tiges porteuses (202) de la partie supérieure (200) étant mobiles en va et vient dans un coussinet de palier respectivement associé pour régler la position de hauteur.

8. Ensemble de protection contre les radiations selon la revendication 7, dans lequel une partie des coussinets de palier sont ajustés dans l'élément de liaison (130) de la partie inférieure (100), et l'autre partie des coussinets de palier (116, 126) dans des premiers (111, 121) et deuxièmes tubes (112, 122).

9. Ensemble de protection contre les radiations selon l'une des revendications 1 à 8, dans lequel l'élément de liaison (201) de préférence cintré en forme d'arc, de la partie supérieure (200), est agencé sur les tiges porteuses (202).

10. Ensemble de protection contre les radiations selon l'une des revendications 7 à 9, dans lequel la partie inférieure (100) comporte, par ailleurs, un dispositif d'immobilisation (133), qui est adapté à fixer au moins l'une des plusieurs tiges porteuses (202) de la partie supérieure (200), de façon à ce que la partie supérieure (200) soit bloquée dans une position insérée dans la partie inférieure (100).

11. Ensemble de protection contre les radiations selon l'une des revendications 1 à 10, dans lequel le deuxième rideau de protection contre les radiations est fermé latéralement par des boutons ou des éléments de fermeture auto-agrippante.

12. Ensemble de protection contre les radiations selon l'une des revendications 1 à 11, dans lequel le deuxième rideau de protection contre les radiations couvre le côté avant de l'ensemble mobile de protection contre les radiations et le côté arrière de l'ensemble mobile de protection contre les radiations, jusqu'à une zone située de 40 cm à 55 cm au-dessus de la structure de châssis de roulement (300), de manière particulièrement préférée jusqu'à 47 cm au-dessus de la structure de châssis de roulement (300).
